(19) **Europäisches Patentamt / European Patent Office / Office européen des brevets**

(11) **EP 4 659 929 A1**

(12) # EUROPEAN PATENT APPLICATION

(43) Date of publication:
**10.12.2025 Bulletin 2025/50**

(21) Application number: 24179888.3

(22) Date of filing: **04.06.2024**

(51) International Patent Classification (IPC):
**B29C 41/14** (2006.01)     **A61B 42/10** (2016.01)
**B29C 41/22** (2006.01)     **A61B 42/30** (2016.01)

(52) Cooperative Patent Classification (CPC):
**B29C 41/14; A61B 42/10; A61B 42/30;**
**B29C 41/22;** B29K 2005/00; B29K 2007/00;
B29K 2009/00; B29K 2075/00; B29K 2105/0064;
B29K 2105/04; B29K 2105/06; B29K 2995/0026;
B29L 2031/4864

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Smarterials Technology GmbH**
**12489 Berlin (DE)**

(72) Inventors:
• **BOTHE, Martin**
  **12489 Berlin (DE)**
• **MIRTSCHIN, Nikolaus**
  **12489 Berlin (DE)**
• **SECKER, Christian**
  **12489 Berlin (DE)**

(74) Representative: **Schulz Junghans**
**Patentanwälte PartGmbB**
**Großbeerenstraße 71**
**10963 Berlin (DE)**

(54) **THREE-LAYERED COVER, PARTICULARLY A SURGICAL GLOVE, AND METHOD OF MAKING SAME**

(57)     An aspect of the invention relates to a multi-layered cover comprising a transparent liquid-impermeable outer layer formed of a first polymer; a liquid-impermeable inner layer formed of a second polymer, and an intermediate layer adhesively joining the outer layer to the inner layer. The intermediate layer is characterized by a thickness of 1 $\mu$m to 100 $\mu$m. The intermediate layer comprises a liquid permeable composition of a third polymer forming an interconnected network of gas filled pores having a diameter of 0.01 - 20 $\mu$m.

Another aspect provides a method for making the cover according to the invention.

**Description**

Field

The present invention relates to a multi-layered cover, particularly a glove for a human hand, composed of at least an outer and inner liquid-impermeable polymer layer, joined by an intermediate layer characterized by gas filled pores having a diameter of 0.01 - 20 $\mu$m. The invention further relates to a method for making such cover. In particular embodiments, the cover is made from one or more polymers, such as polyisoprene.

Background

Surgical gloves are crucial components in the maintenance of aseptic conditions during medical procedures, acting as a barrier between the wearer's hands and the patient, thereby preventing cross-contamination and the transmission of microorganisms. The integrity of these gloves is imperative, and breaches or punctures in their structure can compromise the sterile environment.

Breaches in gloves pose significant risks beyond surgical settings, particularly when handling hazardous substances in various industries such as chemical handling, laboratory work, or in environments dealing with biological agents. The primary concern with breaches in gloves when managing hazardous substances is the potential for direct exposure to these materials.

Moreover, in industrial or research settings, breaches in gloves can result in cross-contamination, not just to the individual handling the substances but also to the surrounding environment, equipment, or other personnel. This can compromise the safety of the workspace, lead to the spread of hazardous materials, and even pose environmental risks if the substances are released outside controlled settings. In situations where sterile or controlled conditions are essential, breaches in gloves may undermine the integrity of experiments, manufacturing processes, or the containment of dangerous agents, leading to potential research or production errors, as well as compromising the safety of workers and the environment.

Breaches in surgical gloves can result from various factors, including manufacturing defects, improper usage, or wear and tear during procedures.

Several methods are employed to detect and address breaches in surgical gloves. Industrial testing on samples is performed according to set standards, e.g. DIN EN 455-1. One of the most common techniques involves visual and tactile inspection, where healthcare professionals visually examine the gloves for any visible damage. However, this method is limited by human perception and may not identify micro-perforations or tiny breaches. Pinholes, such as may occur from needle damage, are especially hard to detect by visual inspection.

Technological advancements have introduced alternative strategies for breach detection. These include water leak tests, air inflation tests, and more sophisticated methods like using dyes or specialized equipment that can detect breaches at a microscopic level.

Based on the above-mentioned state of the art, the objective of the present invention is to provide means and methods to provide a layered cover, particularly a glove, that will allow to readily detect any breach. This objective is attained by the subject-matter of the independent claims of the present specification, with further advantageous embodiments described in the dependent claims, examples, figures and general description of this specification.

Summary of the Invention

A first aspect of the invention relates to a multi-layered cover comprising

    a. a transparent liquid-impermeable outer layer formed of a first polymer;

    b. a liquid-impermeable inner layer formed of a second polymer;

    c. an intermediate layer adhesively joining the outer layer to the inner layer.

The intermediate layer is characterized by a thickness, i.e. distance separating the outer layer from the inner layer, of 1 $\mu$m to 100 $\mu$m. The intermediate layer is permeable to liquids. It comprises, i.e. it is formed, of a composition of a third polymer forming an interconnected network of gas filled pores having a diameter of 0.01 - 20 $\mu$m.

Another aspect of the invention relates to a method for producing a multi-layered cover, comprising the steps:

    a. a cleaned former (mold) is provided in the shape that the multi-layered cover is ultimately going to have. In particular embodiments, this is the shape of a human hand; a subsequent coagulant dipping and drying step;

b. an outer layer polymer characterized by an elongation at break of > 700% (in the finished form) is applied to the former and dried,

c. the former is dipped into an intermediate layer polymer solution characterized by a total solid content (TSC) 2.5% (w/w) to 20% (w/w) (particularly an intermediate layer polymer solution characterized by TSC of 5% (w/w) to 15% (w/w)), wherein the surface temperature of the former is in the range of 50 to 80°C, and the entry/dwell/exit time ranges between 1 to 10s each;

d. the intermediate layer is dried by keeping the surface temperature of the former in the range of 25 °C to 75 °C (particularly in the range of 55°C to 70°C);

e. an inner layer polymer characterized by an elongation at break of > 700% (in the finished form) is applied to the former and dried;

f. the cover is vulcanized in the range of 100 °C to 250 °C for at least 10 minutes.

*Terms and definitions*

*General*

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in organic synthesis, (dip) coating industry).

The term "liquid-impermeable", unless stated otherwise herein, refers to impermeability, defined as a breakthrough time of >10 min against water, sodium hydroxide (40%) or formaldehyde (37%) in methanol (10%), as measured according EN ISO 374-1:2016 + A1:2018 against these chemicals. Breakthrough time in this context is defined as a permeation rate of more than 1.0 $\mu$g cm$^{-2}$ min$^{-1}$.

*Latex:*

In the context of gloves, a "latex" refers to a liquid mixture containing polymer particles. This dispersion is a key component in the manufacturing of latex gloves. It is applied onto glove moulds and then undergoes a drying and curing process to form the final glove.

*Polyisoprene and Natural Rubber Latex:*

From a chemical perspective, polyisoprene is an organic polymer, a type of elastomer, known for its elasticity and resilience. Its chemical formula is $(C_5H_8)_n$, where the "n" represents the number of repeating units. The polymer can occur naturally or be synthetically produced. Natural polyisoprene, often called "natural rubber" and "natural rubber latex", is derived from the latex of rubber trees. Here, the term "polyisoprene" and "polyisoprene latex" refer to the synthetic version, also known as synthetic polyisoprene.

Any patent document cited herein shall be deemed incorporated by reference herein in its entirety.

Detailed Description of the Invention

A first aspect of the invention relates to a multi-layered cover comprising

    a. a transparent liquid-impermeable outer layer formed of a first polymer;

    b. a liquid-impermeable inner layer formed of a second polymer;

    c. an intermediate layer adhesively joining the outer layer to the inner layer.

An alternative formulation of this first aspect may be:
A multi-layered cover, particularly a glove for a human hand, comprising

- a liquid-impermeable outer layer;
- a liquid-impermeable inner layer;
- a porous intermediate layer joining the outer layer to the inner layer,

wherein said intermediate layer forms an interconnected network of pores capable of absorbing liquid, particularly [cj1] through capillary action, if said liquid impermeable outer layer is compromised._

The intermediate layer is characterized by a thickness, i.e. distance separating the outer layer from the inner layer, of 1 $\mu$m to 100 $\mu$m. The intermediate layer comprises, i.e. it is formed, of a composition of a third "intermediate layer" polymer forming an interconnected network of gas filled pores having a diameter of 0.01 - 20 $\mu$m. The intermediate layer is liquid permeable as a result of its porous structure.[PW2][cj3]

The term "intermediate layer" polymer is used as a means of identification only. The "intermediate layer" polymer may indeed in certain embodiments be of the same chemical structure as the first and/or second polymer, but is rendered liquid permeable by its porous structure.

The pores are gas filled pores only before the layer has been breached in contact with a liquid, after which the pores will fill with liquid, changing the optical appearance of the glove, which is a main function of the invention.

In certain embodiments, the intermediate layer is defined as having pores characterized by a diameter of 0.01 - 20 $\mu$m. This of course does not exclude that some pores present in the layer may be outside of this range. In certain embodiments, >50%, >70%, > 80%, >90% or >95% of pores are withing the range of diameter given herein to characterize the pores.[PW4][cj5]

In particular embodiments, the cover provided by the invention is a glove for a human hand.

*Example (transparency measurement)*

Cover transparency was quantified as follows: A sample was placed on top of a microscope slide between a white light source and a photodiode power sensor (Thorlabs S120C used with power and energy interface PM100USB; Thorlabs, NY, USA). The resulting power was recorded with Thorlabs Optical Power Monitor Software as average of approx. 3500 measurement points. The transparency is calculated from the ratio of transmission measured, in relation to the transmission of the microscope slide without cover sample.

In certain embodiments, the outer layer's transparency, determined as given in the example, is $\geq$40%.

In certain embodiments, the outer layer's transparency, determined as given in the example, is $\geq$20% when combined with an inner layer having a distinctly different colour, for example wherein the outer layer is green, and the inner layer is red.

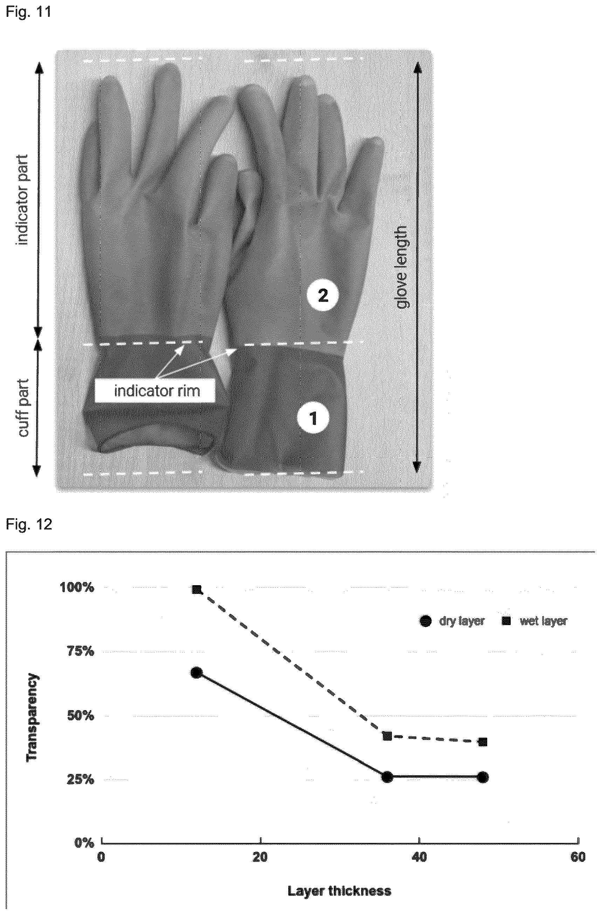

In certain embodiments, the cover is formed as a glove to cover a human hand, having a finger section, a middle hand section and a cuff section (see Fig. 11). In certain particular embodiments thereof, the transparency in the cuff section ranges from 0-40%, and the transparency in the palm- and/or finger sections (indicator part) ranges from 0 to 20%. The transparency may be increased significantly when the glove is breached and the intermediate layer is wetted, i.e. the indicator registers. The inventors have found a relative increase of the transparency of between 40 to 60% upon wetting for a green or blue embodiment of the glove.

The inventors analyzed the transparency of the intermediate layer separated from the outer and inner layer, i.e. without the outer and inner layer present. When dried on top of a cover slip, and then the layer is wetted with water, the transparency increases by 40-60% compared to dry conditions. See Fig. 12.

In certain embodiments, the pores of the multi-layered cover according to the invention are characterized a diameter of 0.1 to 15 $\mu$m, particularly by a diameter of 0.5 $\mu$m - 10 $\mu$m.

Diameter values given in this specification are determined by scanning electron microscopy unless stated otherwise; specifically by analyzing scanning electron microscopy images performed using the software imaged (version 1.53a; https://imaqej.nih.gov/ij/; citation: Schneider, C. A., Rasband, W. S., & Eliceiri, K. W. (2012). NIH Image to imaged: 25 years of image analysis. Nature Methods, 9(7), 671-675. doi:10.1038/nmeth.2089). Thus, image size has to be calibrated; a threshold and segmentation applied to the image to distinguish cavities from the background; cavities selected and its selected size measured by the software.

Protective gloves are typically considered liquid-impermeable to protect the wearer from liquids or chemicals. Surgical gloves from natural rubber latex for example are considered to protect the wearer from water, sodium hydroxide (40%) and formaldehyde (37%) in methanol (10%), among others, when measuring the permeability following EN ISO 3741:2016 + A1:2018 (or ASTM F739) based on their high breakthrough times against these chemicals.

Outer layer / inner layer:

In embodiments where the multi-layered cover is used as a glove, the cover/glove is made by dipping, and thus the total thickness of the layers varies greatly from cuff to finger. Typical glove thicknesses in the palm start at 180 $\mu$m (usually used in microsurgery) through intermediate stages to up to 340 $\mu$m (usually used in orthopaedics). In practice, gloves are often worn twice and thus a factor of 2 can be added to determine what thickness a user may be used to. The glove for "general surgery" is in the 220-260 $\mu$m range.

The inner/outer layer should be at least > 40 $\mu$m to include gloves outside the operating area. In certain embodiments aimed at use in surgery, the layers are each $\geq$ 70 $\mu$m in thickness.

In certain embodiments, the outer layer's thickness is at least 10% greater than the inner layer's thickness. This can be advantageous, as the outer layer is subjected to greater stress due to mechanical loading, abrasion, material fatigue etc. Therefore, embodiments in which the outer layer is thicker than the inner tend to cause the buffer/indicator to strike later.

In certain embodiments, the inner layer's thickness is at least 10% greater than the outer layer's thickness. Embodiments in which the inner layer is thicker than the inner tend to cause the buffer/indicator to strike earlier and therefore could provide a higher safety.

In particular embodiments, the outer layer and the inner layer are each at least 40 $\mu$m in thickness.

In particular embodiments, the outer layer and the inner layer are each at least 70 $\mu$m in thickness.

In particular embodiments, the outer layer and the inner layer are each from 40 to 150 $\mu$m in thickness.

In particular embodiments, the intermediate layer is 20-40[NM6] $\mu$m in thickness.

*Material:*

The outer and inner layer may be made of the same or different materials. In general, a variety of materials lend themselves to the practicing of the invention.

In particular embodiments, the outer layer and the inner layer are made from a material independently selected from natural rubber latex, polyisoprene, polychloroprene, nitrile and polyurethane.

In more particular embodiments, the outer layer and the inner layer are made from a material independently selected from natural rubber latex and polyisoprene.

In particular embodiments, the outer layer and the inner layer are made from the same material.

Natural rubber latex is characterized by very good mechanical properties and comfort of wearing, economical cost, user acceptance and ease of use. The material, however, cannot be used by all potential users, as natural rubber latex is associated with allergic reactions to latex proteins experienced by some individuals.

In certain embodiments, the outer layer essentially consists of polymer, particularly natural rubber latex or synthetic polyisoprene. In certain embodiments, the inner layer essentially consists of polymer, particularly natural rubber latex or synthetic polyisoprene. In certain embodiments, both the outer and the inner layer essentially consist of latex, particularly natural rubber latex.

In certain embodiments, the outer layer essentially consists of polyisoprene. In certain embodiments, the inner layer essentially consists of polyisoprene. In certain embodiments, both the outer and the inner layer essentially consist of polyisoprene. Synthetic polyisoprene is characterized by very good mechanical properties and comfort of wearing, however requires sophisticated manufacturing processes that are costly.

In certain embodiments, the outer layer essentially consists of polychloroprene (neoprene). In certain embodiments, the inner layer essentially consists of polychloroprene. In certain embodiments, both the outer and the inner layer essentially consist of polychloroprene. Polychloroprene material has high chemical resistance, good mechanical properties and comfort of wearing, but again is costly to make.

In certain embodiments, the outer layer essentially consists of nitrile. In certain embodiments, the inner layer essentially consists of nitrile. In certain embodiments, both the outer and the inner layer essentially consist of nitrile, a material of high chemical resistance, which is relatively cheap. Users, however, report moderate mechanical properties and less comfort of wearing and decreased tactile sensitivity compared to natural rubber latex gloves.

In certain embodiments, the outer layer essentially consists of polyurethane. In certain embodiments, the inner layer essentially consists of polyurethane. In certain embodiments, both the outer and the inner layer essentially consist of polyurethane, a material of versatile mechanical properties, which can be submitted to material recycling. It is not, however, as resistant to organic chemicals, exemplified by ethanol, as the other materials described herein.

In certain embodiments, the outer layer and the inner layer are made from different material.

In particular embodiments, the inner layer is made of a material selected from polyisoprene, polychloroprene, nitrile and polyurethane, and the outer layer is made of natural rubber latex.

The inventors contemplate hybrid designs, for example by providing an inner, non-allergy-associated synthetic layer and an outer natural rubber latex layer conferring optimal tactile and lower cost.

*Transparency of the layers:*

The outer layer needs to be characterized by a transparency that is detectable to the human eye under conditions of use in order to allow detection of breach, one of the key features of the layer system provided herein.

The inner layer does not necessarily need to be transparent, but can also be transparent to a certain extent, as it always ends up resting on the user's skin and thus always appears darker. The layers can be essentially of the same transparency and color, as long as the outer layer is sufficiently transparent to allow detection of breach by the change in optical properties of the system.

A good contrast between inner and outer layer helps in this regard. Michelson contrast is widely recognized as a measure of contrast. The Michelson contrast is defined as

$$\frac{I_{\max} - I_{\min}}{I_{\max} + I_{\min}},$$

with I representing the highest and lowest luminance. The luminance can be correlated to grayscale values, which can be easily determined from images using the software Imaged (Schneider et al. ibid.).

For breach detection sufficient change in color is beneficial. Therefore, the contrast between of the undamaged and damaged color of the multi-layered cover should exceed 5%.

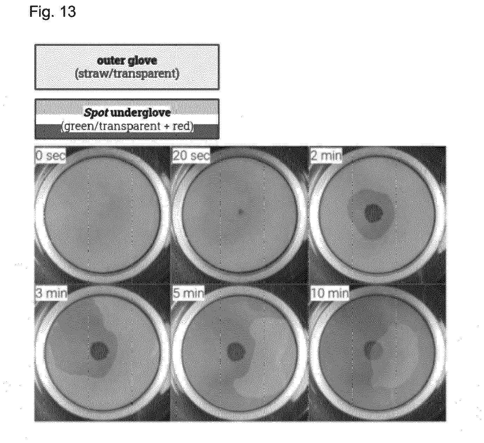

Additionally, it is further possible to put a second glove (overglove) on top of the multi-layer glove disclosed herein (which then is designated "underglove"). This allows the user to distinguish whether the multi-layer underglove or the simpler (more transparent) overglove is perforated. To optimize the effect, the colouring of the multi-layer would need to be distinct from the overglove, rather be red (inner layer) and green (outer layer) (see Fig. 13).

<u>Intermediate layer:</u>

*Intermediate layer structure*

The intermediate layer is formed of a liquid permeable composition of a third polymer forming an interconnected network of gas filled pores having a diameter of 0.01 - 20 $\mu$m. The polymer forming the intermediate layer can be the same as the outer (first polymer) or inner (second polymer) layer, or can be different from one or both of the outer and inner layers' polymers.

The intermediate layer is in adhesive connection to the outer and to the inner layer.

The adhesion of the layers can be quantified using peel tests: sample strips with a width of 6 mm are punched out of the multi-layer. To prevent strong elongation of the sample during testing, it was glued to a support tape from both sides. One end of the "type T" sample was mounted in the upper grip of a universal testing machine and the other end in the lower grip. The peel test was conducted at 500 mm/min. The peel force is defined as the average force value to peel the rectangular sample over a length of 30 mm. Average values from at least 5 samples should be considered.

Herein, the layers are considered to be adhesively bonded to each other when reaching a minimum peel force higher than 1.2 N/(6 mm).

In certain embodiments, the intermediate layer is characterized by its content of solid to pores, i.e. its density. One way of defining this is by measuring the cavity area, for example by 2D-analysis of a SEM image over 10 μm × 30 μm.

Porosity can be described by the pore size distribution and their connectivity, e.g. by the pore/cavity area fraction. The cavity area fraction of the intermediate layer needs to range between 5-90 %, particularly from 10-60%.

Cavity area fraction can be analyzed from a cross-section SEM image of the multi-layer. Therefore, imaged can be used to process the image; apply a threshold and analyze the cavities. The area fraction of the cavities divided by the total area of interest of the intermediate layer provides the cavity area fraction. At least an area of interest of 10 μm × 30 μm has to be analyzed.

The intermediate layer is characterized by interconnected cavities that are formed (defined) by the polymer composition.

In certain embodiments, the third polymer composition comprises a foamed polymer.

Foamed polymer: the intermediate layer's polymer composition is designed in a way that it creates an interconnected network. This network can be understood as a network of polymer fibres and, optionally, particles, to prevent disintegration; likewise, it provides the interconnection of pores to enable the spreading of liquid upon breach. The interconnected network may be formed via a reaction during the vulcanization process, for example evaporation of remaining solvent/continuous phase in the polymer composition resulting in a crosslinked foamed polymer. Alternatively, chemical decomposition of reactants evolving gas products may be used to generate the pores.

In certain embodiments, the intermediate layer's composition of a third polymer comprises a thermoplastic. In particular embodiments, said liquid permeable composition of a third polymer comprises 10% to 70% (w/w). In more particular embodiments, said liquid permeable composition of a third polymer comprises 20% to 60% (w/w) of a thermoplastic.

**Thermoplastics** for practicing the invention include, but are not limited to the group consisting of: polyacrylate, polyurethane, polyvinyl chloride (PVC), styrene-ethylene-butylene-styrene (SEBS), thermoplastic olefin-elastomer (TPO), ethylene-vinyl acetate, acrylonitrile butadiene styrene (ABS), polystyrene, polyethylene, polypropylene, and mixtures of the preceding polymers.

In certain embodiments, said composition of an intermediate layer polymer (third polymer) comprises an elastomer. In particular embodiments, said liquid permeable composition of a third polymer comprises 5% to 60% (w/w), In more particular embodiments, said liquid permeable composition of a third polymer comprises 10% to 50% (w/w) of an elastomer.

**Elastomers** for practicing the invention include, but are not limited to the group consisting of: styrene-butadiene rubber (SBR), polybutadiene (BR), polychloroprene (Neoprene), ethylene propylene diene monomer rubber (EPDM), silicone rubber, polyisoprene (from natural and/or synthetic sources), thermoplastic elastomers (TPE) such as, but not limited to, thermoplastic polyurethane (TPU), styrenic block copolymers (SBC), thermoplastic vulcanizates (TPV), nitrile rubber, butyl rubber, fluoroelastomers, chlorosulfonated polyethylene (CSM), acrylate rubber (ACM), ethylen-propylene rubber (EPM/EPDM).

In certain embodiments, said composition of an intermediate layer polymer (third polymer) comprises at least one (one, or two or three or more) polymer selected from the group of polyacrylate, polyurethane, styrene-butadiene rubber (SBR), polybutadiene (BR), polychloroprene, ethylene propylene diene monomer rubber (EPDM), silicone rubber, polyisoprene (from natural and / or synthetic source), thermoplastic elastomers (TPE), styrol-ethylen-butylen-styrol (SEBS), thermoplastic olefin elastomer (TPO), ethylen vinylacetate, nitril rubber, polyvinylchloride.

In certain embodiments, said composition of an intermediate layer polymer (third polymer) comprises a viscosity enhancing agent. In particular embodiments, the viscosity enhancing agent is present at a content of <8% (w/w). In more particular embodiments, the viscosity enhancing agent is present at a content of 0.5 to 5.0% (w/w). These figures refer to the dry polymer weight (not the suspension employed to make the layer).

In particular embodiments, the viscosity agent is a salt of a fatty acid. In more particular embodiments, the viscosity agent is sodium stearate.

In particular embodiments, said composition of an intermediate layer polymer (third polymer) comprises a polymeric viscosity agent (a viscosity enhancer).

In particular embodiments, said composition of an intermediate layer polymer (third polymer) comprises a polysaccharide as a viscosity enhancer.

Adding a polysaccharide as additive to improve the rheological properties of the layer increases its viscosity and thus improves gel stability (i.e., how well a freshly dipped dip coating solution remains in place after dipping), which has a direct impact on the coating quality, especially homogeneity and layer thickness distribution. In addition, adding the polysaccharide used also results in an increase in tack (tackiness), which also has a positive effect on the layer adhesion, resulting in improved composite material.

Viscosity enhancers for practicing the invention are exemplified, but not limited to, polysaccharides selected from the group that consists of starch, guar gum, xanthan gum, cellulose derivatives (e.g. methylcellulose and hydroxypropyl cellulose), agar-agar, carrageenan, pectin, locust bean gum. This material is used to adjust the dipping viscosity of the suspension. In certain embodiments, the polysaccharide is used in a range between ca. 0.1 and 15% (w/w). These figures refer to the dry polymer weight (not the suspension employed to make the layer).

In certain particular embodiments, the viscosity enhancer is sodium stearate at a concentration of 4.9% (w/w). In certain particular embodiments, the viscosity enhancer is Xanthan at a concentration of 2.1% (w/w). In certain particular embodiments, the viscosity enhancer is Guar at a concentration of 2.8% (w/w).

Composite in this context refers to a material composed of more than one polymer. Adding polyurethane to the intermediate layer's composition, for example, increases adhesion of the layers.

One non-limiting example of a composite intermediate layer material can be built by dip coating into an aqueous suspension of modified silica particles (as spacer for creating cavities) (7.2 wt%), polyester urethane as binder / mechanical mediation (1.1 wt%), inner/outer layer compound (3.6 wt%), xanthan gum as thickener (0.25 wt%), ammonia or potassium hydroxide to adjust pH and water. After drying this composite intermediate layer, it is composed of modified silica particles (59.3 wt%), polyester urethane (8.9 wt%), inner/outer layer compound (29.7 wt%) and xanthan gum (2.1 wt%).

In certain embodiments, polyurethane binders are mixed (e.g. high and medium elongation at break) to better mediate between the mechanical properties of the inorganic and organic materials of the intermediate layer.

In certain embodiments, the polymer composition of a third polymer from which the intermediate layer is made, is a polymer composite.

*Intermediate comprising particles*

The intermediate layer's polymer may additionally comprise a filler. Said filler can fulfill various tasks such as cost reduction, reinforcement or addition of properties the matrix would not have on its own.

In certain embodiments, the intermediate layer additionally comprises a filler.

A filler can be a particle or fibre. It can range from fully crystalline to completely amorphous, it can be organic or inorganic and it can even be of the same chemical nature as the matrix material if it holds a different property or structure such as a fibre.

For most embodiments, the filler is particles. The particles are very stiff/hardly stretchable. Without wanting to be bound by theory, the inventors assume that their effect is based on the fact that the particle structure ensures that the film is not tightly packed, but that there is space for cavities, this effect being determined by their size. Silica is a particularly advantageous material as silica has an affinity for water/hydrophilic due to its chemical structure and due to their ability to be chemically modified (by e.g. silanisation). This is good for processing/manufacturing, indicator effect and "buffering effect" (liquid uptake).

In certain embodiments, the intermediate layer additionally comprises particles.

Particles can be of one or a mixture of various shapes ranging from spherical to fibreous material, defined by its aspect ratio. They can be condensed or porous, defined by its surface area. They can be organic or inorganic and range from fully crystalline to fully amorphous.

In particular embodiments, said particles are characterized by a mean diameter of $\leq 100\ \mu m$.

One exemplary set of particle properties, in this case for silica particles, is given in the following table:

**Particle diameter (nm)**

| Mean: | 111 (Std Dev=22.6) | d50: | 107 | | |
| Mode: | 98 | d10: | 87 | d90: | 142 |
| Maximum: | 273 | d90/d10: | 1.63 | | |
| Minimum: | 67 | Span: | 0.51 | | |

The elasticity of the intermediate layer is adjusted to be in between the particles (elongation at break < 5%) and the top and bottom layer (typically > 1000% elongation at break). Therefore, the polymer composition employed for making the intermediate layer is selected in a way that the individual components making up the respective layers have a gradient of elasticity.

In certain embodiments, the particles are functionalized to confer a water/octanol distribution of 0-5 P if the multi-layered cover is to detect breach of a hydrophilic solution.

In certain embodiments, the particles are functionalized to confer a water/octanol distribution of >5 P if the multi-layered cover is to detect breach of a hydrophobic solution.

In certain embodiments, a particle content in the intermediate layer is in the range of 5% to 90% (w/w), particularly 40% to 80% (w/w).

This refers to the proportion of particles to the total mass of the intermediate layer.

*Intermediate layer emergent behaviour*

In certain embodiments, the intermediate layer is optically opaque or non-transparent when the interconnected cavities are filled with gas, and wherein the intermediate layer has a reduced opacity when the interconnected cavities are filled with a liquid.

In certain embodiments, the intermediate layer is characterized by an opacity ≥80% when the interconnected cavities are filled with gas, and wherein the intermediate layer's is <30% when the interconnected cavities are filled with a liquid.

Where mentioned herein and unless stated otherwise, opacity was determined using reference opacities: The colour of the suspension on a reference black background and the colour on a reference white background is determined and compared. This simple setup can be measured with a camera. White light is used.

In some embodiments, the interconnected cavities are capable of taking up a liquid resulting in a liquid-filled area of >1 mm$^2$ after 1 min in case that the inner or the outer layer is damaged (for example with a liquid-permeable hole). See Example 8 for a detailed description of the measurement.

To determine whether this criterion is fulfilled, the layer system is punctured with an 18G needle, placed in water under slight just enough tension to assure that the layer is straight in two dimensions, and observed over time with a camera at 90° to the surface. The area at the defined time is then determined from the images.

In certain embodiments, the intermediate layer is capable of taking up a liquid from outside of the outer layer or from inside of the inner layer, particularly the intermediate layer is capable of taking up ≥5 µl of a liquid.

The layer system:

The multi-layered cover according to the invention may in certain embodiments be characterized by an elongation at break of 200% - 2000%. In particular embodiments, the elongation at break ranges from 400% - 1500%. In more particular embodiments, the elongation at break ranges from 600 - 1300%. This parameter was measured according to ASTM D412 using a punched specimen (die C or a die D) and an extensometer for strain measurement.

The multi-layered cover according to the invention may in certain embodiments be characterized by a thickness of the intermediate layer of 10 µm to 60 µm. In particular embodiments, the thickness of the intermediate layer may range from 20 µm to 40 µm.

In certain embodiments, the multi-layered cover (inner, intermediate and outer layer together) is characterized by a thickness of 80 to 400 µm. In particular embodiments, the thickness may range from 140 to 350 µm. Where the cover is a glove, this thickness is measured in the palm of the glove.

Specific embodiments of the examples:

In particular embodiments, the intermediate layer comprises particles characterized by a size of 10 nm to 10 µm, particularly a size of 30 nm to 800 nm, more particularly silica particles of a size of 30 nm to 800 nm.

In particular embodiments, the intermediate layer comprises polyisoprene as a first intermediate layer polymer component, which is of the same material as the outer and/or the inner layer.

In particular embodiments, the intermediate layer comprises a second intermediate layer polymer component characterized by an elongation at break of >500% and/or a tensile strength of >10 MPa, particularly polyurethane.

In particular embodiments, the intermediate layer comprises a polysaccharide, particularly xanthene.

In more specific embodiments, the multi-layered cover according to the invention comprises an intermediate layer comprises silica particles of a size of 30 nm to 800 nm, the outer and/or inner layer are made of polyisoprene, the intermediate layer comprises polyisoprene as a first intermediate layer polymer component and polyurethane as a second intermediate layer polymer component, and the intermediate layer comprises a polysaccharide in a range between ca. 0.1 and 15% (w/w).

In even more specific embodiments thereof, the intermediate layer comprises -60% (w/w) of particles.

In yet even more specific embodiments thereof, the intermediate layer comprises -30% (w/w) of the first intermediate layer polymer.

In even more specific embodiments thereof, the intermediate layer comprises -5% (w/w) of the second intermediate layer polymer.

In even more specific embodiments thereof, the intermediate layer comprises -3% (w/w) of a third intermediate layer polymer.

In even more specific embodiments thereof, the intermediate layer comprises -3% (w/w) of the polysaccharide.

Again, viscosity enhancers for practicing the invention are exemplified, but not limited to, polysaccharides selected from the group that consists of starch, guar gum, xanthan gum, cellulose derivatives (e.g. methylcellulose and hydroxypropyl cellulose), agar-agar, carrageenan, pectin, locust bean gum. This material is used to adjust the dipping viscosity of the suspension. In certain embodiments, the polysaccharide is used in a range between ca. 0.1 and 15%.

In certain particular embodiments, the viscosity enhancer is Xanthan at a concentration of 2.1% (w/w). In certain particular embodiments, the viscosity enhancer is Guar at a concentration of 2.8 (w/w).

*Method of producing the layer*

Another aspect of the invention relates to a method for producing a multi-layered cover, comprising the steps:

g. A cleaned former is provided in the shape that the multi-layered cover is ultimately going to have. In particular embodiments, this is the shape of a human hand.

h. In a coagulant dipping and drying step, the mold (former) is essentially just wetted with a salt, which later causes the latex to precipitate and the film to form, and a "former release agent", to facilitate separating the glove from the mold well in the subsequent release step.

i. In an outer layer dipping and drying step, an outer layer polymer characterized by an elongation at break of > 700% (in the finished form) is applied to the former and dried.

j. In an intermediate layer dipping step, the former is dipped into an aqueous intermediate layer polymer solution characterized by a total solid content (TSC) 5% (w/w) to 20% (w/w), wherein the surface temperature of the former is in the range of 25 to 80 °C, particularly in the range of 55 to 75°C, entry/dwell/exit time ranges between 1 to 20s each.

k. In an intermediate layer drying step, the surface temperature of the former is in the range of 30 °C to 75 °C.

l. In an inner layer dipping and drying step, an inner layer polymer characterized by an elongation at break of > 700% (in the finished form) is applied to the former and dried.

m. In a vulcanization step, the cover is vulcanized in the range of 100 °C to 250 °C, particularly 110 °C to 140°C for at least 10 minutes.

One last step is a stripping step that turns the glove inside-out, leaving the outer layer on the outside of the glove.

In particular embodiments, the outer layer polymer and the inner layer polymer used in the method essentially consist of polyisoprene.

In particular embodiments, the intermediate layer polymer solution used in the method according to the invention comprises

- a polymer characterized by an elongation at break of > 500%, and

- particles characterized by a size of 10 nm to 10 $\mu$m, particularly a size of 30 nm to 800 nm.

Yet another aspect of the invention relates to a multi-layered cover obtained by the method according to the invention.

The invention further encompasses the following items:

1. A multi-layered cover, particularly a glove for a human hand, comprising

a. a liquid-impermeable outer layer;

b. a liquid-impermeable inner layer;

c. an intermediate layer joining the outer layer to the inner layer, said intermediate layer being characterized by a thickness of 1 $\mu$m to 100 $\mu$m, said intermediate layer comprising a composition of an intermediate layer polymer forming an interconnected network of pores.

2. The multi-layered cover according to item 1, wherein the pores are characterized by a diameter of 0.01 - 20 $\mu$m.

3. The multi-layered cover according to item 1, wherein the pores are characterized by a diameter of 0.1 to 15 $\mu$m.

4. The multi-layered cover according to item 1, wherein the pores are characterized by a diameter 0.5 $\mu$m - 10 $\mu$m.

5. The multi-layered cover according to any one of the preceding items, wherein the outer layer and the inner layer are each at least 40 $\mu$m in thickness.

6. The multi-layered cover according to any one of the preceding items, wherein the outer layer and the inner layer are each at least 70 $\mu$m in thickness.

7. The multi-layered cover according to any one of the preceding items, wherein the outer layer and the inner layer are each from 40 to 150 $\mu$m in thickness.

8. The multi-layered cover according to any one of the preceding items, wherein the intermediate layer is 20-40 $\mu$m in thickness.

9. The multi-layered cover according to any one of the preceding items, wherein the outer layer is made from a material selected from natural rubber latex, polyisoprene, polychloroprene, nitrile and polyurethane.

10. The multi-layered cover according to any one of the preceding items, wherein the inner layer is made from a material independently selected from natural rubber latex, polyisoprene, polychloroprene, nitrile and polyurethane.

11. The multi-layered cover according to any one of the preceding items, wherein the inner layer is made from a material selected from natural rubber latex and polyisoprene.

12. The multi-layered cover according to any one of the preceding items, wherein the outer layer is made from a material selected from natural rubber latex and polyisoprene.

13. The multi-layered cover according to any one of the preceding items, wherein the outer layer and the inner layer are made from the same material.

14. The multi-layered cover according to any one of the preceding items, wherein the outer layer and the inner layer are made from different material.

15. The multi-layered cover according to item 14, wherein the inner layer is made of a material selected from polyisoprene, polychloroprene, nitrile and polyurethane, and the outer layer is made of natural rubber latex.

16. The multi-layered cover according to any one of the preceding items, wherein said composition of an intermediate layer polymer is a foamed polymer.

17. The multi-layered cover according to any one of the preceding items, wherein said composition of an intermediate layer polymer comprises a thermoplastic.

18. The multi-layered cover according to item 17, wherein said composition of an intermediate layer polymer comprises 10% to 70% (w/w) of a thermoplastic.

19. The multi-layered cover according to item 17, wherein said composition of an intermediate layer polymer comprises 20% to 60% (w/w) of a thermoplastic.

20. The multi-layered cover according to any one of the preceding items, wherein said composition of an intermediate layer polymer comprises an elastomer.

21. The multi-layered cover according to item 20, wherein said composition of an intermediate layer polymer comprises 5% to 60% (w/w) of an elastomer..

22. The multi-layered cover according to item 20, wherein said composition of an intermediate layer polymer comprises 10% to 50% (w/w) of an elastomer.

23. The multi-layered cover according to any one of the preceding items, wherein said composition of an intermediate layer polymer comprises at least one polymer selected from the group of polyacrylate, polyurethane, styrene-butadiene rubber (SBR), polybutadiene (BR), polychloroprene, ethylene propylene diene monomer rubber (EPDM), silicone rubber, polyisoprene (from natural and / or synthetic source), thermoplastic elastomers (TPE), styrol-ethylen-butylen-styrol (SEBS), thermoplastic olefin elastomer (TPO), ethylen vinylacetate, nitril rubber, polyvinylchloride.

24. The multi-layered cover according to any one of the preceding items, wherein said composition of an intermediate layer polymer comprises a viscosity agent.

25. The multi-layered cover according to item 24, wherein said composition of an intermediate layer polymer comprises a viscosity agent at a content of <1% (w/w).

26. The multi-layered cover according to item 24, wherein said composition of an intermediate layer polymer comprises a viscosity agent at a content of 0.1 to 0.5% (w/w).

27. The multi-layered cover according to any one of the preceding items 24 to 26, wherein the viscosity agent is a salt of a fatty acid.

28. The multi-layered cover according to any one of the preceding items 24 to 26, wherein the viscosity agent is sodium stearate.

29. The multi-layered cover according to any one of the preceding items, wherein said composition of an intermediate layer polymer comprises a polysaccharide.

30. The multi-layered cover according to any one of the preceding items, wherein the composition of an intermediate layer polymer is a polymer composite.

31. The multi-layered cover according to any one of the preceding items, wherein the intermediate layer additionally comprises a filler.

32. The multi-layered cover according to any one of the preceding items, wherein the intermediate layer additionally comprises particles.

33. The multi-layered cover according to item 32, wherein said particles are characterized by a mean diameter of $\leq 100$ $\mu$m.

34. The multi-layered cover according to item 32 or 33, wherein said particles are functionalized to confer a water/octanol distribution of 0-5 P.

35. The multi-layered cover according to item 32 or 33, wherein said particles are functionalized to confer a water/octanol distribution of >5 P.

36. The multi-layered cover according to item 32 to 35, wherein a particle content in the intermediate layer is in the range of 5% to 90% (w/w), particularly 40% to 80% (w/w).

37. The multi-layered cover according to any one of the preceding items, wherein the intermediate layer is optically opaque when the interconnected cavities are filled with gas, and wherein the intermediate layer has a reduced opacity when the interconnected cavities are filled with a liquid.

38. The multi-layered cover according to any one of the preceding items, wherein the intermediate layer is characterized by an opacity $\geq 80\%$ when the interconnected cavities are filled with gas, and wherein the intermediate layer's is <30% when the interconnected cavities are filled with a liquid.

39. The multi-layered cover according to any one of the preceding items, wherein the interconnected cavities are capable of taking up a liquid resulting in a liquid-filled area of >1 mm$^2$ after 1 min in case that the inner or the outer layer is damaged.

40. The multi-layered cover according to item 39, wherein the intermediate layer is capable of taking up a liquid from outside of the outer layer or from inside of the inner layer.

41. The multi-layered cover according to item 39, wherein the intermediate layer is capable of taking up $\geq 5$ $\mu$l of a liquid.

42. The multi-layered cover according to any one of the preceding items, characterized by a relative increase of the transparency of the multi-layered cover of greater than 20%.

43. The multi-layered cover according to any one of the preceding items, characterized characterized by a relative increase of the transparency of the multi-layered cover of between 40 to 60% when the interconnected cavities are filled with a liquid.

44. The multi-layered cover according to any one of the preceding items, wherein said multi-layer is characterized by an elongation at break of 200% - 2000%.

45. The multi-layered cover according to any one of the preceding items, wherein said multi-layer is characterized by an elongation at break of 400% - 1500%.

46. The multi-layered cover according to any one of the preceding items, wherein said multi-layer is characterized by an elongation at break of 600 - 1300%.

47. The multi-layered cover according to any one of the preceding items, wherein the intermediate layer is characterized by a thickness of 10 $\mu$m to 60 $\mu$m.

48. The multi-layered cover according to any one of the preceding items, wherein the intermediate layer is characterized by a thickness of 20 $\mu$m to 40 $\mu$m.

49. The multi-layered cover according to any one of the preceding items, wherein the multi-layered cover is characterized by a thickness of 80 to 400 $\mu$m.

50. The multi-layered cover according to any one of the preceding items, wherein the multi-layered cover is characterized by a thickness of 140 to 350 $\mu$m.

51. The multi-layered cover according to any one of the preceding items, wherein the intermediate layer comprises:

   a. particles characterized by a size of 10 nm to 10 $\mu$m;

   b. a first intermediate layer polymer component, which is of the same material as the outer and/or the inner layer;

   c. a second intermediate layer polymer component characterized by an elongation at break of >500% and/or a tensile strength of >10 MPa;

   d. optionally, a polysaccharide, particularly xanthene.

52. The multi-layered cover according to item 51, wherein the intermediate layer comprises particles characterized by a size of 30 nm to 800 nm.

53. The multi-layered cover according to item 51 or 52, wherein the intermediate layer comprises silica particles.

54. The multi-layered cover according to any one of the preceding items 51 to 53, wherein polyisoprene is said first intermediate layer polymer component.

55. The multi-layered cover according to any one of the preceding items 51 to 53, wherein the second intermediate layer polymer component is polyurethane.

56. The multi-layered cover according to any one of items 51 to 55, wherein said intermediate layer comprises -60% (w/w) of particles.

57. The multi-layered cover according to any one of items 51 to 56, wherein said intermediate layer comprises -30% (w/w) of the first intermediate layer polymer.

58. The multi-layered cover according to any one of items 51 to 57, wherein said intermediate layer comprises -5% (w/w) of the second intermediate layer polymer.

59. The multi-layered cover according to any one of items 51 to 58, wherein said intermediate layer comprises -3% (w/w) of a third intermediate layer polymer.

60. The multi-layered cover according to any one of items 51 to 59, wherein said intermediate layer comprises -3% (w/w) of the polysaccharide.

61. A method for producing a multi-layered cover, wherein said method comprises the steps:

   a. providing a former in a shape of the multi-layered cover (particularly in a shape of a human hand);

   b. a coagulant dipping and drying step;

   c. an outer layer dipping and drying step; wherein an outer layer polymer characterized by an elongation at break of > 700% is applied to the former and dried;

   d. an intermediate layer dipping step, wherein the former is dipped into an intermediate layer polymer solution characterized by a total solid content (TSC) 5% (w/w) to 20% (w/w), wherein the surface temperature of the former is in the range of 25 to 80°C, particularly in the range of 30 to 75°C, entry/dwell/exit time ranges between 1 to 10s each;

   e. an intermediate layer drying step; wherein the surface temperature of the former is in the range of 30 °C to 75 °C;

   f. an inner layer dipping and drying step; wherein an inner layer polymer characterized by an elongation at break of > 700% is applied to the former and dried;

   g. a vulcanization step, conducted in the range of 100 °C to 250 °C, particularly 110°C to 140°C for at least 10 minutes.

62. The method according to item 61, wherein the outer layer polymer and the inner layer polymer essentially consist of polyisoprene.

63. The method according to item 61 or 62, wherein the intermediate layer polymer solution comprises

   - a polymer characterized by an elongation at break of > 500%, and

   - particles characterized by a size of 10 nm to 10 $\mu$m, particularly a size of 30 nm to 800 nm.

64. A multi-layered cover obtained by the method according to any one of items 61 to 63.

Wherever alternatives for single separable features are laid out herein as "embodiments" or "items", it is to be understood that such alternatives may be combined freely to form discrete embodiments of the invention disclosed herein.
The invention is further illustrated by the following examples and figures, from which further embodiments and advantages can be drawn. These examples are meant to illustrate the invention but not to limit its scope.

*Description of the Figures*

Fig. 1:   shows a multilayer according with the invention comprising an outer layer (1), intermediate layer with thickness t and interconnected cavities (2) and inner layer (3) and below: damaged outer layer (4) with cavities in intermediate layer filled with a liquid (5), which spreading can be described by a diameter d (6). Therefrom, the buffer uptake can be calculated.

Fig. 2   shows an SEM image of the intermediate layer of the layered structure prepared in Example 2.

Fig. 3   shows the results of a breach experiment with the layer prepared in Example 2.

Fig. 4   shows an SEM image of the intermediate layer of the layered structure prepared in Example 3.

Fig. 5    shows the results of a breach experiment with the layer prepared in Example 3.

Fig. 6    shows an SEM image of the intermediate layer of the layered structure prepared in Example 4.

Fig. 7    shows the results of a breach experiment with the layer prepared in Example 4.

Fig. 8    shows an SEM image of the intermediate layer of the layered structure prepared in Example 5.

Fig. 9    shows the results of a breach experiment with the layer prepared in Example 5.

Fig. 10   Liquid-filled area/indicator measuring device with evaluation unit (left) and sectional view of the sample holding device (right). 1: screen; 2: height adjustment; 3: camera; 4: sample holding device; 5: liquid chamber; 6: screw clamp; 7: mandrel; 8: body; 9: adjusting screw.

Fig. 11   shows an exemplary glove having distinct transparencies in the cuff and palm/finger sections: the cuff section (1) has a transparency of 0-40% (specifically 28% in the embodiment shown), the palm/finger ("indicator") section (2) has a lower transparency, here 0-20% (specifically 11% in the embodiment shown for the dry glove, and 18% after wetting by breach).

Fig. 12   shows transparency for dry and wet intermediate layers as a function of intermediate layer thickness [$\mu$m] measured on top of microscope slide (without inner and outer layer).

Examples

In the following, different non-limiting embodiments of the invention are explained in more detail to give examples and show the variety of appearance of the multi-layered cover, and particularly of the intermediate layer.

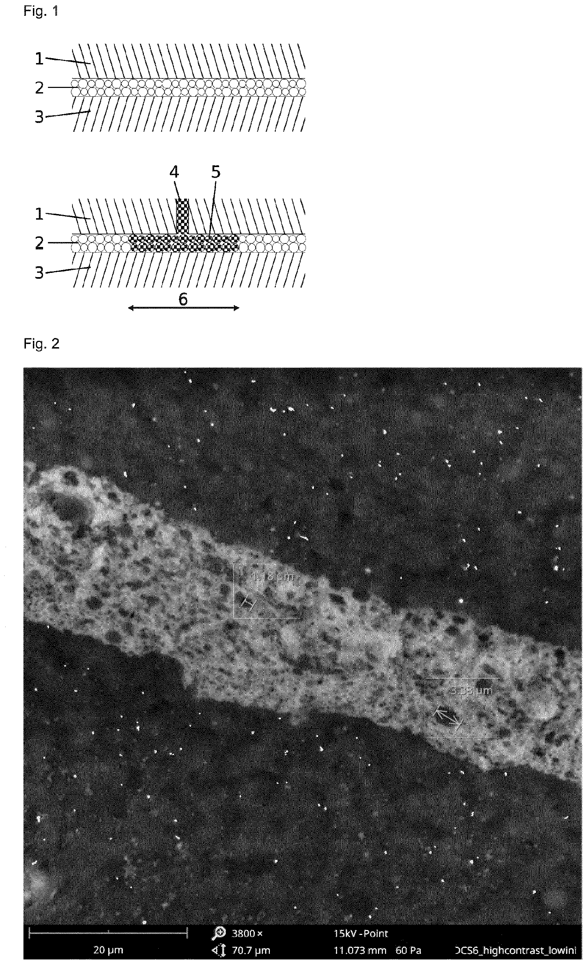

*Example 1: Liquid collecting intermediate layer (see Fig. 1)*

Buffer uptake of multi-layered sample can be measured by weighing:

-    take a sample with diameter 40 mm and weight => ca. 310 mg

-    apply indicator/liquid-filled area test for certain time and weight again => ca. 316 mg

=> ca. 6 mg removed surrounding water

=> equals **0.006 cm³** (density of water is 1 g/cm³)

=> verification/plausibility test:

1. after liquid was collected in the intermediate layer, a spot with a radius $r$ of 1.2 cm was discolored

2. volume can be calculated accordingly: $V = (\pi\, r^2) * h$, where $h$ is the effective total height of the cavities, hence: $h$ = layer-thickness * cavity-area-fraction

3. A layer-thickness of 39 $\mu$m = 0.0039 cm and cavity-area-fraction = 0.32 was determined

4. Volume can be calculated: **0.0057 cm³** (calculation similar to test above)

*Example 2: Manufacturing a liquid collecting intermediate layer*

*Materials preparation:*

*- Outer layer:*

Polyisoprene dispersion sourced from Cariflex (diluted to 45% TSC), equipped with Booster mix Bostex 862 (5% PHR) and Manawet 172 (0.75% PHR), both sourced from Akron Dispersions, Ohio. pH is adjusted. (It can be (prevulcanized) natural rubber compounding, nitrile compounding as well).

*- Inner layer:*

Polyisoprene dispersion sourced from Cariflex (diluted to 45% TSC), equipped with Booster mix Bostex 862 (5% PHR) and Manawet 172 (0.75% PHR), both sourced from Akron Dispersions, Ohio. Additionally Farsperse Blue MPL-FAW sourced from Farben Technique was added (0.25% PHR). pH is adjusted. (It can be (prevulcanized) natural rubber compounding, nitrile compounding as well).

*- Coagulant:*

Aqueous solution of calcium nitrate (18% wt) and calcium stearate (4% wt) and wetting agent. (calcium stearate can be replaced with calcium carbonate)

*- Intermediate layer:*

Aqueous solution for dip coating comprising the following:

| Chemical | Function | TSC (wt%) | CAD (wt%) |
|---|---|---|---|
| Modified particles | Spacer / creating cavities | 7.20 | 59.3 |
| Polyurethane 1 | Binder / mechanical mediation | 0.72 | 5.9 |
| Polyurethane 2 | Binder / mechanical mediation | 0.36 | 3.0 |
| Outer/inner layer compound (polyisoprene latex) | Binder / mechanical mediation | 3.61 | 29.7 |
| Xanthane gum | Viscosity agent | 0.25 | 2.1 |
| Water | for dip coating (added to match 100%) | | 0 |
| Ammonia | Adjustment of pH | - | - |
| TSC: Total solid content; CAD: Content after drying | | | |

*Manufacturing procedure:*

A method for producing a multi-layered cover, such as a glove, wherein said method comprises of the following steps:

1. A former cleaning step, thorough scrub with soapy water and preheating step;
2. coagulant dipping step, dried up to a surface temperature of 65°C;
3. an outer layer of polyisoprene latex dipping step, wherein the surface temperature of the former needs to be at 55°C when dried at an 85°C, entry/dwell/exit time 1/3/1 seconds at 45% TSC;
4. an outer layer drying step; wherein the surface temperature of the former needs to be 65°C;
5. an intermediate layer dipping step, wherein the surface temperature of the former needs to be 55°C more entry/dwell/exit time 1/3/1 seconds based on 12.1% TSC;
6. an intermediate layer drying step, wherein the surface temperature of the former needs to be 65°C;
7. an inner layer dipping step, wherein the surface temperature of the former needs to be in the range of 55°C, entry/dwell/exit time 1/10/1 seconds at a TSC of 45%;
8. an inner layer drying step, wherein the surface temperature of the former needs to be 65°C;
9. a leaching step for 2 min in deionized water at 65°C
10. a pre-drying step at 85°C for 15 min
11. a vulcanization step, at 130°C for 15 min
12. a post leaching step for 2 min in deionized water at 65°C
13. stripping the multi-layered cover, so that the (first dipped) outer layer is outside and the (last dipped) inner layer is inside the cover

Steps such as polymer coating, washing chlorination, sterilization etc. can optionally be added.

*Result: (see Fig. 2)*

SEM image shows interconnected cavities, dominant size range is between 0.5 - 3.0 $\mu$m (not excluding cavities in the size above and beyond that range) (Fig. 2)

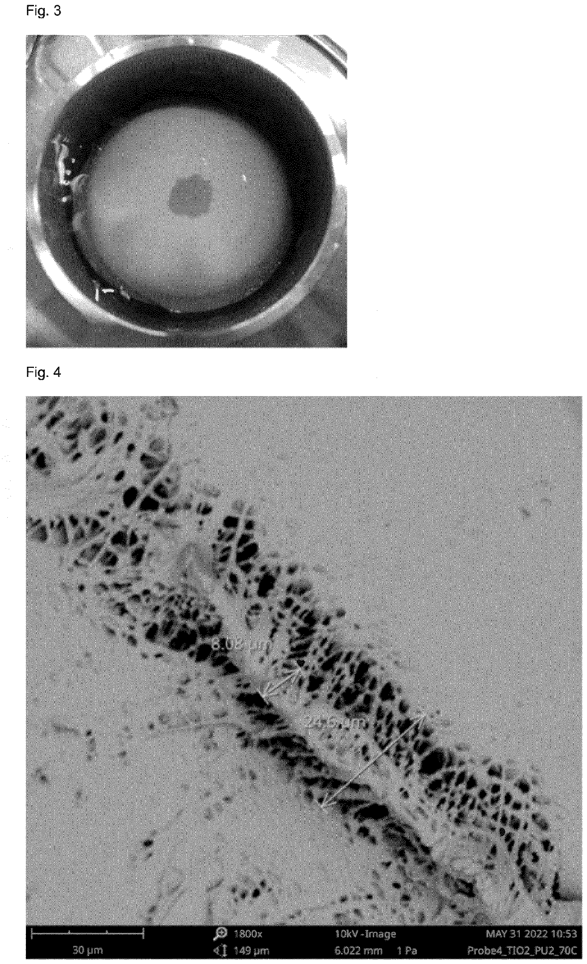

The intermediate layer is continuous and cavities are evenly distributed between adjacent liquid-impermeable inner and outer layers. Image analysis revealed a cavity area fraction in the intermediate layer of ca. 27%. This setup results in 2.9 N per 6 mm peel resistance and a small to medium liquid-filled area (0.1 - 0.4 cm$^2$), see Fig. 3.

*Example 3: Adjusting intermediate layer performance*

The performance of the intermediate layer can be adjusted by tuning the microstructure e.g. by the interlayer composition (other materials and manufacturing remain the same).

*Materials preparation (interlayer):*

Aqueous solution for dip coating comprising the following:

| Chemical | Function | TSC (wt%) | CAD (wt%) |
|---|---|---|---|
| Modified particles | Spacer / creating cavities | 5.00 | 69.9 |
| Polyurethane | Binder / mechanical mediation | 1.80 | 25.2 |
| Sodium stearate | Viscosity agent | 0.35 | 4.90 |
| Water | for dip coating (added to match 100%) | | |
| Ammonia | Adjustment of pH | - | - |
| TSC: Total solid content; CAD: Content after drying | | | |

*Results:*

An SEM image (Fig. 4) of the prepared cover's intermediate layer structure revealed interconnected cavities, their dominant size range being between 3.0 - 5.0 $\mu$m (not excluding cavities in the size above and beyond that range), but with more cavities at the interface of the inner layer.

The intermediate layer is composed of a condensed middle part and expanded interfacial region next to the adjacent liquid-impermeable inner and outer layer. The expanded region is connected to both the condensed middle layer and the adjacent layers, the intermediate layer is relatively evenly spread in.

The image analysis gave a cavity area fraction in the intermediate layer of ca. 40%.

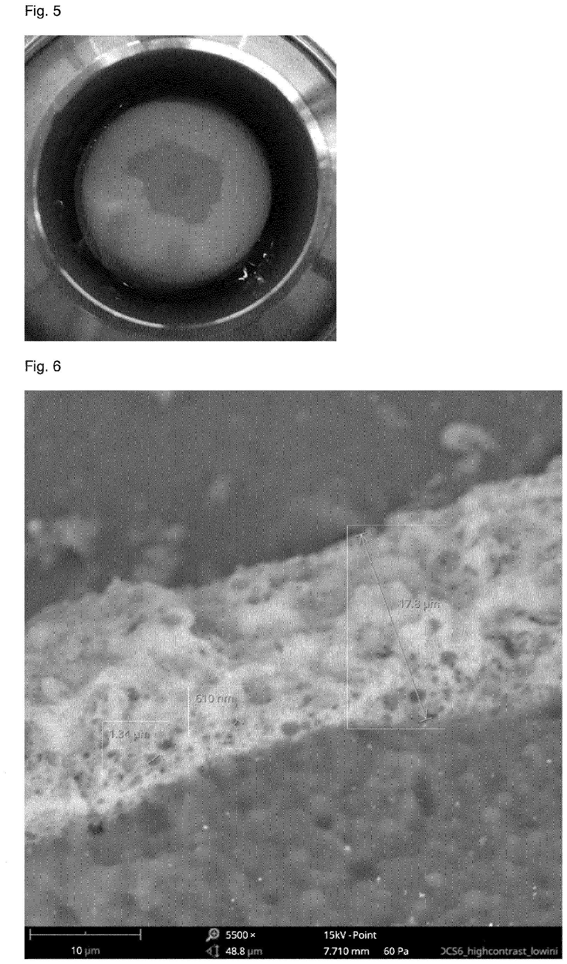

This setup results in 1.5 N per 6 mm peel resistance and a large liquid-filled area (0.9 - 1.3 cm$^2$), see Fig. 5.

*Example 4: Influence of drying conditions*

The manufacturing procedure and especially the drying conditions significantly affect the resulting buffer performance. The materials and process followed Example 2, but differed in drying conditions.

*Influence of drying conditions I*

An exemplary multi-layered cover was obtained by the following steps:

1. a former cleaning step, thorough scrub with soapy water;
2. coagulant step, (calcium nitrate, calcium stearate in aqueous solution, 1.08 density) dried up to a surface temperature of 65°C;
3. an outer layer of polyisoprene latex dipping step, wherein the surface temperature of the former needs to be at 55°C when dried at an 85°C, entry/dwell/exit time 1/3/1 seconds at 45% TSC;
4. an outer layer drying step; wherein the surface temperature of the former needs to be 65°C;
5. an intermediate layer dipping step, wherein the surface temperature of the former needs to be 55°C more entry/dwell/exit time 1/3/1 seconds based on 12.1% TSC;
6. an intermediate layer drying step; wherein the surface temperature of the former needs to be 65°C;

7. an inner layer dipping step; wherein the surface temperature of the former needs to be in the range of 55°C, entry/dwell/exit time 1/10/1 seconds at a TSC of 45% ;

8. an inner layer drying step, wherein the surface temperature of the former needs to be 65°C;

9. a leaching step for 2 min in deionized water at 65°C;

10. a final drying step at 85°C for 15 min.

**Results:**

The sample was not exposed to any temperature above 85°C - most water has not been boiled, accordingly, building of cavities through gas evolving from water is suppressed and the water remains captured inside.

An SEM image of the prepared cover's intermediate layer structure revealed interconnected cavities, their dominant size range being between 0.3 - 1.5 $\mu$m (not excluding cavities in the size above and beyond that range) (see Fig. 6).

The intermediate layer is composed of a condensed middle part and expanded interfacial region next to the adjacent liquid-impermeable inner and outer layer. The expanded regions are larger than in the structure of Example 2 and relatively evenly spread to both sides of the condensed middle layer, still both sides of the are linked to the middle and the adjacent layers.

The image analysis gave a cavity area fraction in the intermediate layer of ca. 11%.

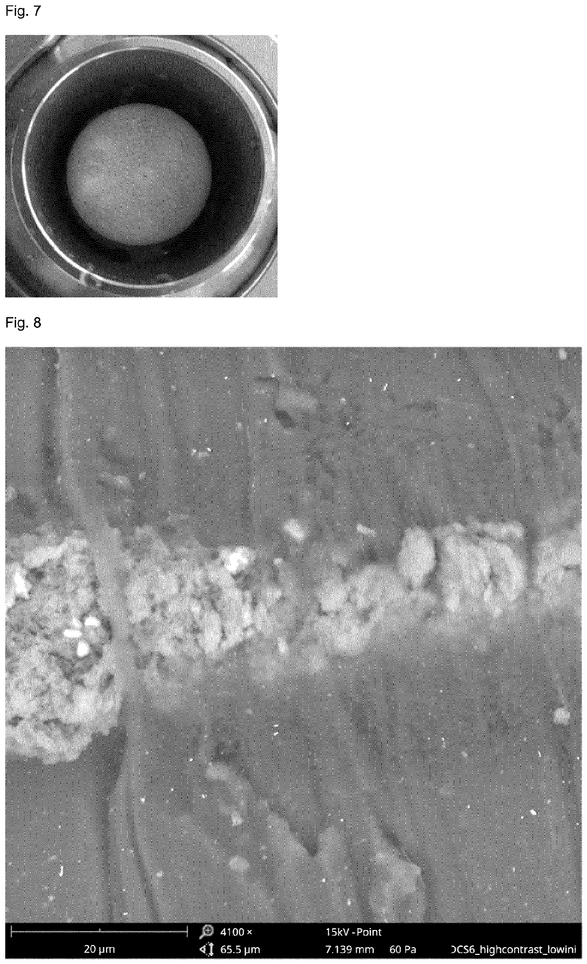

This setup results in 2.0 N per 6 mm peel resistance and a very small / no liquid-filled area (< 0.1 cm$^2$), see Fig. 7.

*Example 5: Influence of drying conditions II*

An exemplary multi-layered cover was obtained by the following steps:

1. A former cleaning step, thorough scrub with soapy water;

2. A coagulant step, (calcium nitrate, calcium stearate in aqueous solution, 1.08 density) dried up to a surface temperature of 65°C 130°C oven temperature;

3. an outer layer of polyisoprene latex dipping step, wherein the surface temperature of the former needs to be at 55°C, entry/dwell/exit time 1/3/1 seconds at 45% TSC;

4. an outer layer drying step; wherein the surface temperature of the former needs to be 65°C 130°C oven temperature;

5. an intermediate layer dipping step, wherein the surface temperature of the former needs to be 55°C more entry/dwell/exit time 1/3/1 seconds based on 12.1% TSC;

6. an intermediate layer drying step; wherein the surface temperature of the former needs to be 65°C at 130°C oven temperature;

7. an inner layer dipping step; wherein the surface temperature of the former needs to be in the range of 55°C, entry/dwell/exit time 1/10/1 seconds at a TSC of 45% ;

8. an inner layer drying step, wherein the surface temperature of the former needs to be 65°C at 130°C oven temperature;

9. a leaching step for 2 min in deionized water at 65°C;

10. a predring step at 85°C for 15 min;

11. a vulcanization step, at 130°C for 15 min;

12. a post leaching step for 2 min in deionized water at 65°C;

**Results:**

The sample has been exposed to temperatures above 100°C - most water has been boiled too early, accordingly, unable to build cavities.

SEM revealed interconnected cavities, the dominant size range of which is between < 0.5 $\mu$m (not excluding cavities in the size above and beyond that range) (see Fig. 8).

The intermediate layer is composed of a condensed middle part and expanded interfacial region next to the adjacent liquid-impermeable inner and outer layer. The expanded regions are larger than in the structure of Example 2 and relatively evenly spread to both sides of the condensed middle layer, still both sides of the are linked to the middle and the adjacent layers. Image analysis gave a cavity area fraction in the intermediate layer of ca. <5%

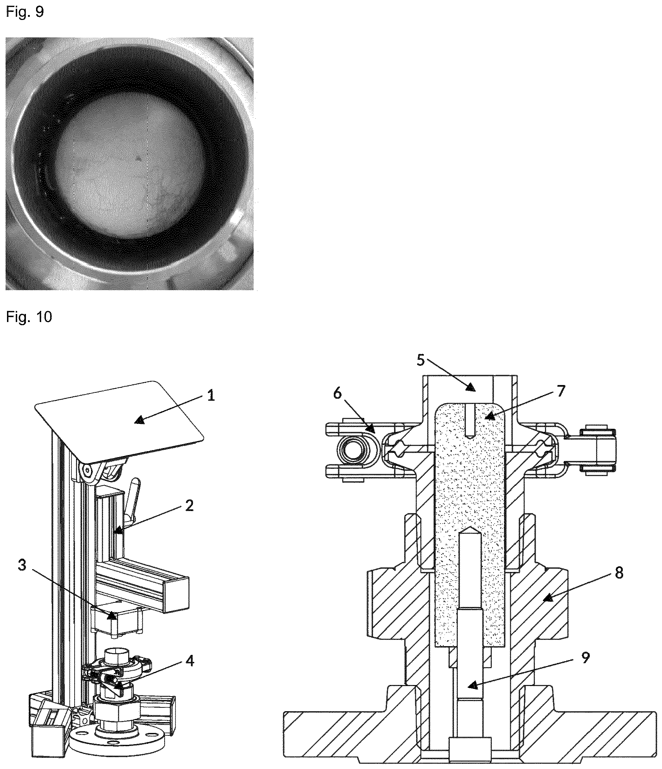

This setup results in 3.6 N per 6 mm peel resistance and very small / no liquid-filled area (< 0.1 cm$^2$), see Fig. 9.

*Example 6: Opacity measurement*

The intermediate layer as provided in Example 2 was coated with a thickness of ~40 μm on top of a reference (black/white) opacity card. The opacity can be measured with a spectrophotometer or estimated by taking camera images and analyzing the grayscale values of the image. Firstly, images were captured with the intermediate layer on top of the black and white part of the opacity card and the corresponding grayscale values $G_{black, I}$ and $G_{white, I}$ synthesized by image analysis. Secondly, a water droplet was put on top of the intermediate layer and the corresponding grayscale values $G_{black, II}$ and $G_{white, II}$ analyzed.

The opacity can be defined as the grayscale value of a film on a black surface to that of a film on a white surface:

$$\text{opacity in } \% = G_{black} / G_{white} * 100\%$$

The opacity of the neat film was -90%, which was reduced after exposed to water to -44%.

*Methods*

*Example 7: Peel resistance // peel strength*

- standardized measurement for layer adhesion
- based on **T peel test** with rectangular samples (6 mm width; >60 mm length) cut with a punch
- samples were supported by application of double sided PSA tape to avoid elongation during testing
- the force during delaminating the parallel part of the sample is analyzed and related to the cross section width of 6 mm

*Example 8: Liquid-filled area or indicator testing (refer to Fig. 10)*

- sample of 40 mm circle is cut using a cutting die or a punch
- the sample is clamped between the main body (8) and the liquid chamber (5) using a screw clamp (6)
- the sample is pre-tensioned using an adjusting screw (9) and a mandrel (7)
- a new and unused needle (Sterican® / B. Braun Melsungen AG: G18 × 1 ½", ø1,20 × 40 mm, Ref. 4665120) is used to place a vertical stitch in the middle of the sample
- the camera (3) is focused once on the sample using a height adjustment (2)
- the perforation can be seen in the middle of the screen (1), a recording is started and the liquid chamber (5) is filled to the top with water
- the software measures the area of the perforation by color analysis

*Example 9: SEM // image analysis*

Samples were cut at low temperature (< -40 °C) to prevent smearing of the rubber layers.

*Buffer function // water uptake by weighing*

- sample of 40 mm circle is cut using a cutting die or a punch
- sample weight w1 is recorded
- a new and unused needle (Sterican® / B. Braun Melsungen AG: G18 × 1 ½", ø1,20 × 40 mm, Ref. 4665120) is used to place a vertical stitch in the middle of the sample
- a water droplet is put on top of the stitch to initiate water uptake of the intermediate layer
- after 3 min, the remaining water on top of the inner and/or outer layer is removed with a lint free cloth
- sample weight w2 is recorded
- the difference w2 - w1 provides the uptake of water of the intermediate layer

*Cited references:*

All scientific publications and patent documents cited in the present specification are incorporated by reference herein.

**Claims**

1.  A multi-layered cover, particularly a glove for a human hand, comprising

    a. a liquid-impermeable outer layer;
    b. a liquid-impermeable inner layer;
    c. an intermediate layer joining the outer layer to the inner layer, said intermediate layer being **characterized by** a thickness of 1 μm to 100 μm, said intermediate layer comprising a composition of an intermediate layer polymer forming an interconnected network of pores.

2.  The multi-layered cover according to claim 1, wherein the pores are **characterized by** a diameter of 0.01 - 20 μm, particularly by a diameter of 0.1 to 15 μm, more particularly by a diameter of 0.5 μm - 10 μm.

3.  The multi-layered cover according to any one of the preceding claims, wherein said composition of an intermediate layer polymer comprises a thermoplastic, particularly wherein said composition of an intermediate layer polymer comprises 10% to 70% (w/w), more particularly 20% to 60% (w/w) of a thermoplastic.

4.  The multi-layered cover according to any one of the preceding claims, wherein said composition of an intermediate layer polymer comprises an elastomer, particularly wherein said composition of an intermediate layer polymer comprises 5% to 60% (w/w), particularly 10% to 50% (w/w) of an elastomer.

5.  The multi-layered cover according to any one of the preceding claims, wherein said composition of an intermediate layer polymer comprises at least one polymer selected from the group of polyacrylate, polyurethane, styrene-butadiene rubber (SBR), polybutadiene (BR), polychloroprene, ethylene propylene diene monomer rubber (EPDM), silicone rubber, polyisoprene (from natural and / or synthetic source), thermoplastic elastomers (TPE), styrol-ethylen-butylen-styrol (SEBS), thermoplastic olefin elastomer (TPO), ethylen vinylacetate, nitril rubber, polyvinylchloride.

6.  The multi-layered cover according to any one of the preceding claims, wherein said composition of an intermediate layer polymer comprises a polysaccharide.

7.  The multi-layered cover according to any one of the preceding claims, wherein the intermediate layer additionally comprises particles, wherein said particles are **characterized by** a mean diameter of ≤ 100 μm.

8.  The multi-layered cover according to claim 7, wherein said particles are functionalized to confer a water/octanol distribution of 0-5 P.

9.  The multi-layered cover according to any one of the preceding claims, wherein the intermediate layer is optically opaque when the interconnected cavities are filled with gas, and wherein the intermediate layer has a reduced opacity when the interconnected cavities are filled with a liquid.

10. The multi-layered cover according to any one of the preceding claims, wherein the intermediate layer is **characterized by** an opacity ≥80% when the interconnected cavities are filled with gas, and wherein the intermediate layer's is <30% when the interconnected cavities are filled with a liquid.

11. The multi-layered cover according to any one of the preceding claims, wherein the interconnected cavities are capable of taking up a liquid resulting in a liquid-filled area of >1 mm$^2$ after 1 min in case that the inner or the outer layer is damaged.

12. The multi-layered cover according to any one of the preceding claims, **characterized by** a relative increase of the transparency of the multi-layered cover of greater than 20%, particularly **characterized by** a relative increase of the transparency of the multi-layered cover of between 40 to 60% when the interconnected cavities are filled with a liquid.

13. The multi-layered cover according to any one of the preceding claims, wherein the intermediate layer comprises:

   a. particles **characterized by** a size of 10 nm to 10 μm, particularly a size of 30 nm to 800 nm, more particularly silica particles;
   b. a first intermediate layer polymer component, which is of the same material as the outer and/or the inner layer, particularly wherein polyisoprene is said first intermediate layer polymer component;
   c. a second intermediate layer polymer component **characterized by** an elongation at break of >500% and/or a tensile strength of >10 MPa, particularly polyurethane;
   d. optionally, a polysaccharide, particularly xanthene.

14. A method for producing a multi-layered cover, wherein said method comprises the steps:

   a. providing a former in a shape of the multi-layered cover (particularly in a shape of a human hand);
   b. a coagulant dipping and drying step;
   c. an outer layer dipping and drying step; wherein an outer layer polymer **characterized by** an elongation at break of > 700% is applied to the former and dried;
   d. an intermediate layer dipping step, wherein the former is dipped into an intermediate layer polymer solution **characterized by** a total solid content (TSC) 5% (w/w) to 20% (w/w), wherein the surface temperature of the former is in the range of 25 to 80°C, particularly in the range of 30 to 75°C, entry/dwell/exit time ranges between 1 to 10s each;
   e. an intermediate layer drying step; wherein the surface temperature of the former is in the range of 30 °C to 75 °C;
   f. an inner layer dipping and drying step; wherein an inner layer polymer **characterized by** an elongation at break of > 700% is applied to the former and dried;
   g. a vulcanization step, conducted in the range of 100 °C to 250 °C, particularly 110°C to 140°C for at least 10 minutes.

15. The method according to claim 14, wherein the outer layer polymer and the inner layer polymer essentially consist of polyisoprene.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Europäisches
Patentamt

European
Patent Office

Office européen
des brevets

# EUROPEAN SEARCH REPORT

**Application Number**

EP 24 17 9888

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/259332 A1 (HASSAN NOORMAN BIN ABU [MY] ET AL) 18 September 2014 (2014-09-18) | 1-7,9-15 | INV. B29C41/14 A61B42/10 B29C41/22 A61B42/30 |
| A | * paragraphs [0039], [0034], [0035], [0048], [0038], [0033], [0026]; claim 10; figures 1,2 * | 8 | |
| X | WO 2012/108907 A1 (ANSELL LTD [AU]; HASSAN NOORMAN BIN ABU [MY] ET AL.) 16 August 2012 (2012-08-16) | 1-7,9-15 | |
| A | * paragraphs [0041], [0042], [0021], [0037] - [0039], [0046]; claim 4; figure 1 * | 8 | |
| X | US 2015/157072 A1 (MEGAT ABDUL AZIZ PUTRI FARIDATUL AKMAR [MY] ET AL) 11 June 2015 (2015-06-11) | 1-7,9-15 | |
| A | * paragraphs [0015], [0031], [0017], [0023]; claims 14,15,5; figure 4 * | 8 | |
| A | WO 2020/130784 A1 (INEO TECH SDN BHD [MY]) 25 June 2020 (2020-06-25) * paragraphs [0030] - [0035] * | 1-15 | TECHNICAL FIELDS SEARCHED (IPC) |
| A | US 2024/081446 A1 (MIRTSCHIN NIKOLAUS [DE] ET AL) 14 March 2024 (2024-03-14) * claims 1,15; figures 2,3 * | 1-15 | B29C A61B B29K B29L A41D C08K |
| A | WO 2007/068873 A1 (REGENT MEDICAL LTD [GB]; PICKARD SIMON [GB]; LEEMING CHRISTINE [GB]) 21 June 2007 (2007-06-21) * claims 1,3,8,10,12; figure 1 * | 1-15 | |
| A | US 5 817 433 A (DARRAS ROBERT [US]) 6 October 1998 (1998-10-06) * claim 1; figure 2 * | 1-15 | |
| A | US 5 549 924 A (SHLENKER ROBIN R T [US] ET AL) 27 August 1996 (1996-08-27) * claims 1,14,17; figure 4 * | 1-15 | |

The present search report has been drawn up for all claims

| Place of search | Date of completion of the search | Examiner |
|---|---|---|
| Munich | 6 November 2024 | Ferrer Santos, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 24 17 9888

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2024

| Patent document cited in search report | | | Publication date | Patent family member(s) | | Publication date |
|---|---|---|---|---|---|---|
| US 2014259332 | A1 | 18-09-2014 | | AU | 2014231759 A1 | 27-08-2015 |
| | | | | CN | 105073420 A | 18-11-2015 |
| | | | | EP | 2969566 A1 | 20-01-2016 |
| | | | | US | 2014259332 A1 | 18-09-2014 |
| | | | | WO | 2014138782 A1 | 18-09-2014 |
| WO 2012108907 | A1 | 16-08-2012 | | US | 2011287553 A1 | 24-11-2011 |
| | | | | WO | 2012108907 A1 | 16-08-2012 |
| US 2015157072 | A1 | 11-06-2015 | | AU | 2014354564 A1 | 26-05-2016 |
| | | | | CN | 206165886 U | 17-05-2017 |
| | | | | EP | 3073851 A1 | 05-10-2016 |
| | | | | US | 2015157072 A1 | 11-06-2015 |
| | | | | WO | 2015077821 A1 | 04-06-2015 |
| WO 2020130784 | A1 | 25-06-2020 | | MY | 195324 A | 13-01-2023 |
| | | | | US | 2022061944 A1 | 03-03-2022 |
| | | | | WO | 2020130784 A1 | 25-06-2020 |
| US 2024081446 | A1 | 14-03-2024 | | NONE | | |
| WO 2007068873 | A1 | 21-06-2007 | | GB | 2433227 A | 20-06-2007 |
| | | | | WO | 2007068873 A1 | 21-06-2007 |
| US 5817433 | A | 06-10-1998 | | US | 5817433 A | 06-10-1998 |
| | | | | US | 6020057 A | 01-02-2000 |
| US 5549924 | A | 27-08-1996 | | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHNEIDER, C. A.** ; **RASBAND, W. S.** ; **ELICEIRI, K. W.** NIH Image to imaged: 25 years of image analysis.. *Nature Methods*, 2012, vol. 9 (7), 671-675